(19) Europäisches Patentamt
European Patent Office
Office européen des brevets

(11) Veröffentlichungsnummer: **0 073 974**
**A2**

# (12) EUROPÄISCHE PATENTANMELDUNG

(21) Anmeldenummer: 82107513.2

(22) Anmeldetag: 18.08.82

(51) Int. Cl.³: A 01 N 43/70
C 07 D 251/50, C 07 D 251/52
C 07 D 251/70
//C07D251/44

(30) Priorität: 29.08.81 DE 3134227
19.05.82 DE 3218966

(43) Veröffentlichungstag der Anmeldung:
16.03.83 Patentblatt 83/11

(84) Benannte Vertragsstaaten:
AT BE CH DE FR GB IT LI NL SE

(71) Anmelder: BAYER AG
Zentralbereich Patente, Marken und Lizenzen
D-5090 Leverkusen 1, Bayerwerk(DE)

(72) Erfinder: Kühle, Engelbert, Dr.
von Bodelschwinghstrasse 42
D-5060 Bergisch-Gladbach 2(DE)

(72) Erfinder: Baasner, Bernd, Dr.
Mozartstrasse 41
D-5090 Leverkusen 1(DE)

(72) Erfinder: Hagemann, Hermann, Dr.
Kandinsky-Strasse 52
D-5090 Leverkusen(DE)

(72) Erfinder: Eue, Ludwig, Dr.
Paul-Klee-Strasse 36
D-5090 Leverkusen(DE)

(72) Erfinder: Schmidt, Robert R., Dr.
Im Waldwinkel 110
D-5060 Bergisch Gladbach 2(DE)

(54) **Fluorhaltige 4,6-Diamino-s-triazine, Verfahren und neue Zwischenprodukte zu ihrer Herstellung und ihre Verwendung als Herbizide.**

(57) Neue, fluorhaltige 4,6-Diamino-s-triazine der allgemeinen Formel

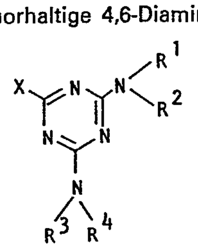

(1),

worin
X für Chlor, Alkoxy mit 1 - 4 C-Atomen, Alkylthio mit 1 - 4 C-Atomen oder den Azidrest ($-N_3$) steht,
$R^1$, $R^2$ und $R^3$ gleich oder verschieden sind und für Wasserstoff und/oder einen gesättigten oder ungesättigten aliphatischen Rest stehen und
$R^4$ für einen Fluoralkyl- oder Fluorchloralkylrest steht,
ein Verfahren und neue Zwischenprodukte zu ihrer Herstellung und ihre Verwendung als Herbizide.

EP 0 073 974 A2

BAYER AKTIENGESELLSCHAFT
Zentralbereich
Patente, Marken und Lizenzen

5090 Leverkusen, Bayerwerk
28.08.81
Bi/bo/c
Ia

Fluorhaltige 4,6-Diamino-s-triazine, Verfahren und neue Zwischenprodukte zu ihrer Herstellung und ihre Verwendung als Herbizide

Die vorliegende Erfindung betrifft neue fluorhaltige s-Triazinderivate, Verfahren und neue Zwischenprodukte zu ihrer Herstellung sowie ihre Verwendung als Herbizide.

Es ist bereits seit langem bekannt, daß bestimmte s-Triazinderivate als Herbizide eingesetzt werden können. Besondere praktische Bedeutung hat z.B. das 2-Chlor-4-ethylamino-6-isopropylamino-s-triazin (Atrazin) erlangt, welches bekanntermaßen für die Unkrautbekämpfung in Maiskulturen verwendet werden kann (vgl. z.B. "Chemie der Pflanzenschutz- und Schädlingsbekämpfungsmittel", Band 5 - Herbizide, herausgegeben von R.Wegler, Springer-Verlag Berlin, Heidelberg, New-York, 1977, Seiten 336 - 352).

Atrazin ist aber gegen hirseartige Gräser, z.B. Echinochloa, Digitaria, Setaria etc. nicht bzw. nicht voll wirksam.

Es wurden nun neue fluorhaltige 4,6-Diamino-s-triazine der allgemeinen Formel

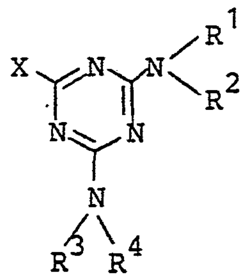

(I),

worin

X für Chlor, Alkoxy mit 1 - 4 C-Atomen, Alkylthio mit 1 - 4 C-Atomen oder den Azidrest ($-N_3$) steht,

$R^1$, $R^2$ und $R^3$ gleich oder verschieden sind und für Wasserstoff und/oder einen gesättigten oder ungesättigten aliphatischen Rest stehen und

$R^4$ für einen Fluoralkyl- oder Fluorchloralkylrest steht,

aufgefunden.

Weiterhin wurde gefunden, daß man die s-Triazinderivate der allgemeinen Formel (I) erhält, wenn man 2,4-Dichlor-6-(fluoralkylamino)-s-triazine der allgemeinen Formel II

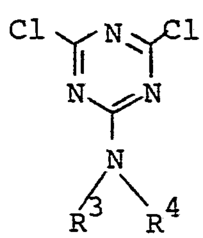

(II),

worin

$R^3$ und $R^4$ die oben angegebene Bedeutung haben,

mit einem Amin der Formel III

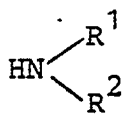

(III),

worin

$R^1$ und $R^2$ die oben angegebene Bedeutung haben,

in Gegenwart eines säurebindenden Mittels und gegebenenfalls in Gegenwart eines Verdünnungsmittels umsetzt (1. Stufe) und die hierbei gebildeten 2-Chlor-4,6-diamino-s-triazine der Formel Ia

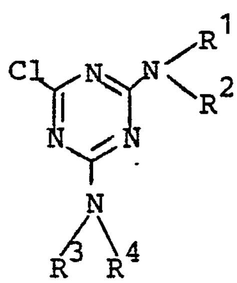

(Ia),

worin

$R^1$, $R^2$, $R^3$ und $R^4$ die oben angegebene Bedeutung haben,

gegebenenfalls mit einem $C_{1-4}$-Alkohol, einem $C_{1-4}$-Mercaptan oder mit Stickstoffwasserstoffsäure ($HN_3$) jeweils

in Salzform oder in Gegenwart eines säurebindenden Mittels und gegebenenfalls in Gegenwart eines Verdünnungsmittels umsetzt (2. Stufe), wobei 2-substituierte 4,6-Diamino-s-triazine der Formel Ib

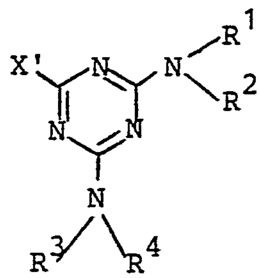

(Ib),

worin

X' für Alkoxy oder Alkylthio mit jeweils 1 - 4 C-Atomen oder für den Azidrest steht und

$R^1$, $R^2$, $R^3$ und $R^4$ die oben angegebene Bedeutung haben,

erhalten werden (Verfahren A).

Außerdem wurde gefunden, daß die neuen fluorhaltigen s-Triazinderivate der allgemeinen Formel (I) starke herbizide Eigenschaften aufweisen.

Überraschenderweise haben die erfindungsgemäßen Wirkstoffe eine bessere selektive Wirkung und ein breiteres Wirkungsspektrum als die vorbekannten Triazine. Insbesondere hat sich gezeigt, daß die erfindungsgemäßen Wirkstoffe gegen hirseartige Gräser erheblich besser wirksam sind als das vorbekannte Atrazin.

Von den erfindungsgemäßen Triazinen der Formel (I) sind bevorzugt diejenigen, in denen X für Chlor, Alkoxy mit 1 - 4 C-Atomen, Alkylthio mit 1 - 4 C-Atomen oder den Azidrest steht, $R^1$, $R^2$ und $R^3$ jeweils unabhängig voneinander für Wasserstoff, Alkyl mit 1 - 6 C-Atomen oder Alkenyl mit 3 - 6 C-Atomen stehen und $R^4$ für einen Fluoralkylrest mit 1 - 8 C-Atomen und 1 - 9 F-Atomen oder einen Fluorchloralkylrest mit 1 - 8 C-Atomen und bis zu insgesamt 9 Fluor- und Chloratomen steht. $R^2$ kann auch für einen der folgenden aliphatischen Reste stehen: Ethylthioethyl, Methoxyethyl, 2-Chlorethyl, 2-Hydroxyethyl, Ethoxycarbonylethyl.

Verwendet man gemäß Verfahren A beispielsweise 2,4-Dichlor-6-(1-methyl-2,2,2-trifluorethylamino)-s-triazin und Ethylamin als Ausgangsstoffe und setzt das hierbei gebildete 2-Chlor-4-ethylamino-6-(1-methyl-2,2,2-trifluorethylamino)-s-triazin weiter mit Natriummethanolat um, so kann der Reaktionsablauf zusammenfassend durch das folgende Formelschema wiedergegeben werden:

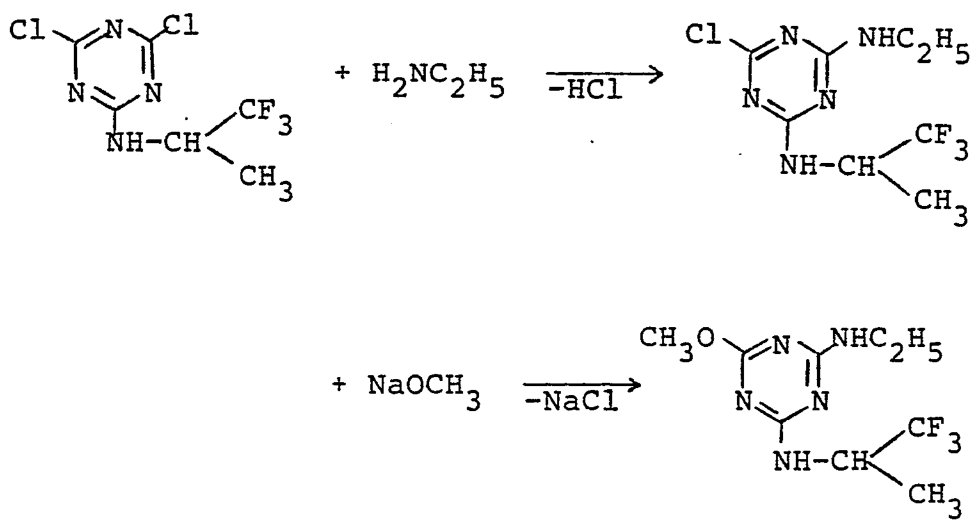

Die nach Verfahren A als Ausgangsverbindungen verwendeten 2,4-Dichlor-6-(fluor-alkylamino)-s-triazine der Formel (II) sind neu. Sie können hergestellt werden, indem man Cyanurchlorid der Formel IV

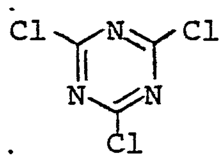

(IV)

mit fluorhaltigen primären und sekundären Aminen der Formel V

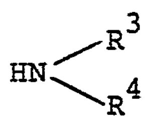

(V)

worin

$R^3$ und $R^4$ die oben angegebene Bedeutung haben,

in Gegenwart eines säurebindenden Mittels und gegebenenfalls in Gegenwart eines Verdünnungsmittels umsetzt (Verfahren B).

Verwendet man gemäß Verfahren B Cyanurchlorid und beispielsweise 1-Methyl-2,2,2-trifluor-ethylamin als Ausgangsstoffe, so läßt sich der Reaktionsablauf durch das folgende Formelschema wiedergeben:

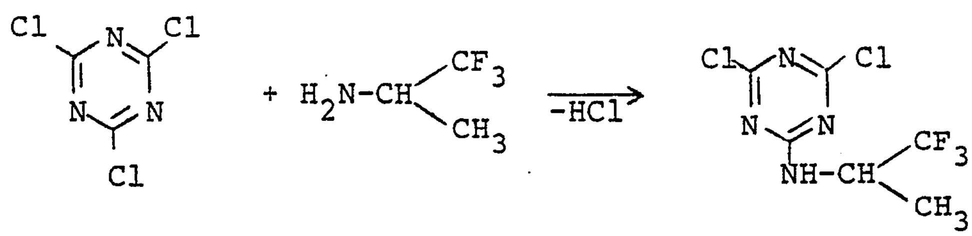

In den Formeln (V) bzw. (II) steht $R^3$ vorzugsweise für Wasserstoff, Alkyl mit 1 - 6 C-Atomen oder Alkenyl mit 3 - 6 C-Atomen. $R^4$ steht in diesen Formeln vorzugsweise für geradkettiges oder verzweigtes Fluoralkyl mit 1 - 8 C-Atomen und 1 - 9 F-Atomen oder für geradkettiges oder verzweigtes Fluorchloralkyl mit 1 - 8 C-Atomen und bis zu insgesamt 9 Fluor- und Chlor-Atomen.

Besonders bevorzugt sind solche Verbindungen der Formeln (V) bzw. (II), in denen $R^4$ für geradkettiges oder verzweigtes Fluoralkyl mit 1 - 6 C-Atomen und 1 - 7 F-Atomen oder für geradkettiges oder verzweigtes Fluorchloralkyl mit 1 - 6 C-Atomen und bis zu insgesamt 7 Fluor- und Chloratomen steht.

Speziell genannt seien solche Verbindungen der Formel (V) bzw. (II), in denen $R^4$ für 2,2,2-Trifluorethyl, Pentafluorethyl, 3,3,3-Trifluorpropyl, 2,2,2-Trifluor-1-methyl-ethyl, 4,4,4-Trifluor-n-butyl, 1-(Trifluormethyl)-n-propyl, 1-(Trifluormethyl)-n-butyl, 1-(Trifluormethyl)-n-pentyl, 1-(Trifluormethyl)-2-methylpropyl, 3-Fluor-3,3-dichlor-n-propyl, 3-Fluor-3-chlor-n-propyl, 2,2-Difluorpropyl oder für 2,3,3,3-Tetrafluor-2-chlor-propyl steht.

0073974

Die nach Verfahren A weiterhin als Ausgangsverbindungen zu verwendenden Amine sind durch Formel (III) allgemein definiert. In dieser Formel stehen $R^1$ und $R^2$ vorzugsweise für Wasserstoff, Alkyl mit 1 - 6 C-Atomen oder Alkenyl mit 3 - 6 C-Atomen. $R^2$ kann auch für einen der folgenden aliphatischen Reste stehen: Ethylthioethyl, Methoxyethyl, 2-Chlorethyl , 2-Hydroxyethyl, Ethoxycarbonylmethyl.

Die Amine der Formel (III) sind bekannt. Geeignete Vertreter sind Ammoniak, Methyl-, Ethyl-, n-Propyl-, Isopropyl-, n-Butyl-, Isobutyl-, tert.-Butyl-, Hexyl-, Allyl-, Diethyl-, Diallyl- und Dibutylamin.

Zur Herstellung von s-Triazinen der Formel (Ib) werden die s-Triazine der Formel (Ia) mit einem $C_{1-4}$-Alkohol, einem $C_{1-4}$-Mercaptan oder mit Stickstoffwasserstoffsäure umgesetzt. In der Formel (Ib) haben $R^1$, $R^2$, $R^3$ und $R^4$ die bereits angegebenen vorzugsweisen Bedeutungen. Geeignete $C_{1-4}$-Alkohole sind z.B. Methanol, Ethanol, n-Propanol, n-Butanol und iso-Butanol. Geeignete $C_{1-4}$-Mercaptane sind z.B. Methyl-, Ethyl-, n-Propyl-, iso-Propyl- und n-Butylmercaptan. Geeignete Salze der genannten Alkohole und Mercaptane sind vorzugsweise die Alkalisalze, insbesondere die Natriumsalze.

Die bei Verfahren B einzusetzenden fluorhaltigen Amine der Formel (V) sind teilweise bekannt (vgl. J. Org. Chem. 24, S 1256 - 1259 (1959); J. Chem. Soc. 1954, S. 366 - 374; J. Org. Chem. 27, S. 1406 - 1409 (1962); J. Med. Chem. 22, S. 1130 - 1133 (1979); Izvest. Akad. Nauk. SSSR Ser. Khim. 1966, S. 1518 - 1923 (engl.); US-PS 3 908 012; US-PS 3 960 949 und DE-OS 2 117 015).

Die bisher noch nicht im einzelnen beschriebenen fluorierten Amine der Formel (V) lassen sich nach im Prinzip bekannten Methoden herstellen.

So erhält man zum Beispiel diejenigen fluorierten Amine der Formel Va

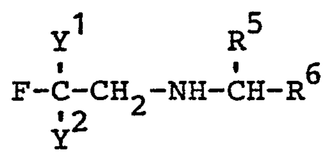 (Va)

in welcher

$Y^1$ für Wasserstoff, Fluor oder Chlor steht,
$Y^2$ für Wasserstoff, Fluor oder Chlor steht,
$R^5$ für Alkyl steht und
$R^6$ für Wasserstoff oder Alkyl steht,

indem man fluorierte Azomethine der Formel VI

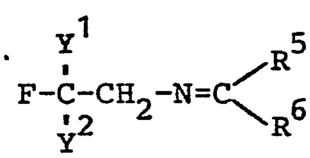 (VI)

in welcher

$Y^1$, $Y^2$, $R^5$ und $R^6$ die oben angegebene Bedeutung haben,

mit Wasserstoff unter einem Druck von 3 bis 15 bar in Gegenwart eines Katalysators, wie Platin auf Kohle,

Palladium auf Kohle oder Raney-Nickel, sowie in Gegenwart eines Verdünnungsmittels, zum Beispiel eines Alkohols wie Methanol oder Ethanol, oder eines Ethers wie Dioxan, bei Temperaturen zwischen 0°C und 60°C, vorzugsweise zwischen 10°C und 50°C hydriert.

In der Formel (VI) steht $Y^1$ vorzugsweise für Wasserstoff, Fluor oder Chlor. $Y^2$ steht ebenfalls vorzugsweise für Wasserstoff, Fluor oder Chlor. $R^5$ steht vorzugsweise für Alkyl mit 1 bis 4 Kohlenstoffatomen, und $R^6$ steht vorzugsweise für Wasserstoff oder Alkyl mit 1 bis 4 Kohlenstoffatomen.

Die bei der Herstellung der fluorierten Amine der Formel (Va) nach dem oben beschriebenen Verfahren als Ausgangsstoffe benötigten Azomethine der Formel (VI) sind bisher noch nicht bekannt. Sie lassen sich jedoch herstellen, indem man Amine der Formel VII

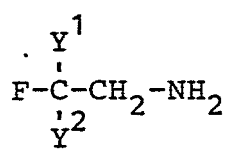

(VII)

in welcher

$Y^1$ und $Y^2$ die oben angegebene Bedeutung haben,

mit Carbonylverbindungen der Formel VIII

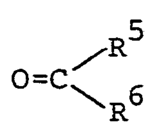

(VIII)

in welcher

$R^5$ und $R^6$ die oben angegebene Bedeutung haben,

gegebenenfalls in Gegenwart eines Verdünnungsmittels, wie Pentan, Hexan, Cyclohexan, Chloroform, Tetrachlorkohlenstoff, Benzol, Toluol, Xylol, Chlorbenzol, Diethylether, Tetrahydrofuran oder Dioxan, bei Temperaturen zwischen -20°C und +60°C, vorzugsweise zwischen 0°C und +40°C umsetzt.

Die bei der Synthese der fluorierten Azomethine der Formel (VI) nach dem obigen Verfahren als Ausgangsstoffe benötigten Amine der Formel (VII) und Carbonylverbindungen der Formel (VIII) sind bekannt oder lassen sich nach im Prinzip bekannten Methoden herstellen (vgl. hierzu DE-OS 30 18 030).

Das als Ausgangsverbindung besonders interessante 1-Methyl-2,2,2-trifluorethylamin kann zweckmäßigerweise ausgehend von 1,1-Difluorpropen über 1-Methyl-2,2,2-trifluornitroethan und dessen katalytische Hydrierung - gemäß folgendem Formelschema - hergestellt werden (vgl. zur Methode: Izvest. Akad. Nauk SSSR, Ser. Khim. 1963, S. 1794 ff (engl.); ferner Tetrahedron 26, S. 5737 - 5743 (1970)):

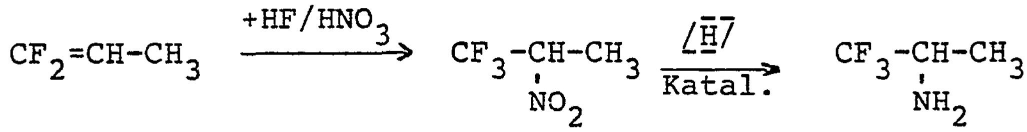

Bei der Durchführung der konjugierten Addition von HF und $HNO_3$ an 1,1-Difluorpropen setzt man die Salpetersäure bevorzugt in äquimolarer Menge ein (wobei ein Überschuß möglich ist ); Fluorwasserstoff wird in überstöchiometrischen Mengen eingesetzt und dient gleichzeitig als Lösungsmittel. Man erhält die Nitroverbindung in Ausbeuten bis zu 86 % der Theorie. - Bei der anschließenden katalytischen Hydrierung können alle üblichen Edelmetallkatalysatoren wie Pd oder Pt, aber auch Raney-Nickel verwendet werden. Ferner kann die Reduktion auch mittels nascierendem Wasserstoff, z.B. mit dem System $Fe/H^+$ - mit oder ohne Zusatz von Basen wie $MgCO_3$ oder $Na_2CO_3$ als Säurefänger - durchgeführt werden. Als Lösungs- bzw. Verdünnungsmittel sind Alkohole wie Methanol oder Ethanol, Ether wie z.B. Diisopropylether sowie Wasser geeignet (vgl. Beispielteil).

Die Reaktionstemperaturen können bei den beiden oben beschriebenen erfindungsgemäßen Verfahren A und B in einem gewissen Bereich variiert werden. Im allgemeinen arbeitet man bei der ersten Stufe von Verfahren A zwischen 0 und 50°C, (vorzugsweise zwischen 20 und 45°C), bei der zweiten Stufe von Verfahren A im allgemeinen zwischen 50 und 150°C (vorzugsweise zwischen 50 und 110°C), und bei Verfahren B im allgemeinen zwischen -10 und +25°C (vorzugsweise zwischen 0 und 20°C).

Die nachfolgenden allgemeinen Angaben betreffend die Molverhältnisse, Verdünnungsmittel, Säurebindemittel und Aufarbeitung beziehen sich jeweils auf die beiden Verfahren A und B.

Bei der Durchführung der erfindungsgemäßen Verfahren setzt man die Ausgangsstoffe und gegebenenfalls die Säurebindemittel in etwa äquimolaren Mengen ein.

Die Umsetzungen werden bevorzugt in Gegenwart eines Verdünnungsmittels durchgeführt. Als Verdünnungsmittel kommen alle inerten organischen Lösungsmittel in Frage. Hierzu gehören vorzugsweise Kohlenwasserstoffe, wie Benzol und Xylol, chlorierte Kohlenwasserstoffe, wie Chlorbenzol und Tetrachlorkohlenstoff; Ketone, wie Aceton und Methylethylketon; Ether, wie Tetrahydrofuran und Dioxan; Amide, wie Dimethylformamid.

Die Umsetzungen können zum Teil auch im wäßrigen Medium durchgeführt werden. Im Falle der Umsetzung von Triazinen der Formel (II) mit Alkoholaten kann als Verdünnungsmittel vorteilhaft der dem Alkoholat zugrundeliegende Alkohol verwendet werden.

Als Säurebinder können alle üblichen Säurebindungsmittel verwendet werden. Hierzu gehören vorzugsweise die tertiären Amine, wie Trimethylamin, Triethylamin und Pyridin, Alkalihydroxide, wie Natriumhydroxid und Kaliumhydroxid sowie auch Alkalicarbonate, wie Natriumcarbonat und Kaliumcarbonat. Bei der ersten Stufe von Verfahren A kann auch ein äquimolarer Überschuß an Amin der Formel (III) als Säurebinder dienen.

Nach einer besonderen Ausführungsform der erfindungsgemäßen Verfahren können Verfahren B und die erste Stufe von Verfahren A als "Eintopfreaktion" durchgeführt werden, wobei die Zwischenprodukte der Formel (II) nicht isoliert zu werden brauchen.

Die Aufarbeitung und Isolierung der Reaktionsprodukte erfolgt in üblicher Weise. Bei Zugabe von Wasser zum Reaktionsgemisch fallen die Reaktionsprodukte im allgemeinen bereits kristallin aus, falls Lösungsmittel verwendet worden sind, die sich mit Wasser mischen.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

<u>Dicotyle Kulturen der Gattungen:</u> Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Verbindungen wirken sowohl gegen dikotyle Pflanzen, insbesondere Sinapis, Chenopodium, Urtica, Stellaria, Galium, Daucus, wie auch gegen monokotyle Pflanzen, insbesondere Hirse-Arten. Gerade in der Wirkung gegen Hirse-Arten, z.B. Echinochloa, Digitaria, Setaria, sind die neuen Wirkstoffe den bekannten herbiziden Triazinen überlegen. Ein selektiver Einsatz der erfindungsgemäßen Wirkstoffe ist in verschiedenen Kulturen möglich, z.B. in Getreide, Baumwolle und Zwiebeln. Die Wirkstoffe sind insbesondere für Mais sehr gut verträglich; sie können daher mit besonderem Vorteil als selektive Maisherbizide eingesetzt werden, wobei sie wegen ihrer wesentlich besseren Wirkung insbesondere gegen hirseartige Schadgräser dem vorbekannten Maisherbizid Atrazin überlegen sind.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sephiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugenden Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methycellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden - z.B. Chloracetaniliden wie Alachlor oder Metolachlor - zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen oder Stäuben.

Die erfindungsgemäßen Wirkstoffe können sowohl vor als auch nach dem Auflaufen der Pflanzen appliziert werden. Sie können auch vor der Saat in den Boden eingearbeitet werden.

Die aufgewandte Wirkstoffmenge kann in größeren Bereichen schwanken. Sie hängt im wesentlichen von der Art des gewünschten Effektes ab. Im allgemeinen liegen die Aufwandmengen zwischen 0,1 und 10 kg Wirkstoff pro ha, vorzugsweise zwischen 0,2 und 5 kg/ha.

Die nachfolgenden Beispiele dienen zur weiteren Erläuterung der Erfindung.

Herstellungsbeispiele

A) Triazine der Formel (Ia) - nach Verfahren A/1. Stufe

Beispiel A-1

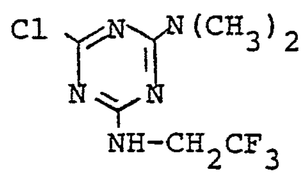

14,8 g (0,06 Mol) 2,4-Dichlor-6-(2,2,2-trifluorethylamino)-s-triazin (Beispiel C-1) werden in 100 ml Aceton gelöst und bei Raumtemperatur tropfenweise mit 12 ml einer etwa 45 %igen wäßrigen Dimethylaminlösung versetzt. Durch Wasserkühlung hält man die Temperatur unter 30°C. Durch weiteren Wasserzusatz fällt man das Reaktionsprodukt aus. Man erhält 14 g (= 93 % d.Th.) 2-Chlor-4-dimethylamino-6-(2,2,2-trifluorethylamino)-s-triazin; Schmelzpunkt 181 - 184°C.

In analoger Weise erhält man die in der nachfolgenden Tabelle 1 aufgeführten Verbindungen der Formel Ia

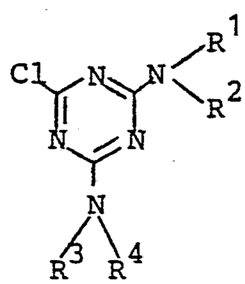

(Ia):

Tabelle 1

| Bsp.-Nr. | $R^1$ | $R^2$ | $R^3$ | $R^4$ | Schmelzpunkt (Brechungsindex $n_D^{20}$) |
|---|---|---|---|---|---|
| A-2 | H | $C_2H_5$ | H | $CF_3CH_2-$ | 244 - 245°C |
| A-3 | H | $C_3H_5$ * | H | $CF_3CH_2-$ | 235 - 236°C |
| A-4 | H | $n-C_3H_7$ | H | $CF_3CH_2-$ | 170 - 171°C |
| A-5 | $C_2H_5$ | $C_2H_5$ | H | $CF_3CH_2-$ | 149°C |
| A-6 | $CH_3$ | $CH_3$ | H | $CH_3CF_2CH_2-$ | 158 - 160°C |
| A-7 | H | $C_2H_5$ | H | $CH_3CF_2CH_2-$ | 217 - 218°C |
| A-8 | H | $i-C_3H_7$ | H | $CH_3CF_2CH_2-$ | 135 - 139°C |
| A-9 | H | $C_3H_5$ * | H | $CH_3CF_2CH_2-$ | 219 - 220°C |
| A-10 | $C_2H_5$ | $C_2H_5$ | H | $CH_3CF_2CH_2-$ | 119°C |
| A-11 | $C_3H_7$ | $C_3H_7$ | H | $CH_3CF_2CH_2-$ | 115°C |
| A-12 | H | $n-C_3H_7$ | $CH_3$ | $CF_3CH_2-$ | 129°C |
| A-13 | H | $CH_3$ | $CH_3$ | $CF_3CH_2-$ | 140°C |
| A-14 | $C_2H_5$ | $C_2H_5$ | $CH_3$ | $CF_3CH_2-$ | (1,4998) |
| A-15 | $CH_3$ | $CH_3$ | $CH_3$ | $CF_3CH_2-$ | 64°C |
| A-16 | H | $i-C_3H_7$ | $CH_3$ | $CF_3CH_2-$ | 74 - 75°C |
| A-17 | H | $C_2H_5$ | $CH_3$ | $CF_3CH_2-$ | 145 - 147°C |
| A-18 | H | $C_3H_5$ * | $CH_3$ | $CF_3CH_2-$ | 118°C |
| A-19 | H | $n-C_3H_7$ | $C_2H_5$ | $CF_3CH_2-$ | 120 - 121°C |
| A-20 | H | $CH_3$ | $C_2H_5$ | $CF_3CH_2-$ | 134 - 137°C |

Tabelle 1 (Fortsetzung)

| Bsp.-Nr. | $R^1$ | $R^2$ | $R^3$ | $R^4$ | Schmelzpunkt (Brechungsindex $n_D^{20}$) |
|---|---|---|---|---|---|
| A-21 | $C_2H_5$ | $C_2H_5$ | $C_2H_5$ | $CF_3CH_2-$ | (1,4922) |
| A-22 | $CH_3$ | $CH_3$ | $C_2H_5$ | $CF_3CH_2-$ | 59°C |
| A-23 | H | $i-C_3H_7$ | $C_2H_5$ | $CF_3CH_2-$ | 67 - 68°C |
| A-24 | H | $C_2H_5$ | $C_2H_5$ | $CF_3CH_2-$ | 132°C |
| A-25 | H | $C_3H_5$ * | $C_2H_5$ | $CF_3CH_2-$ | 116°C |
| A-26 | H | $i-C_3H_7$ | $C_4H_9$ | $CF_3CH_2-$ | 68 - 72°C |
| A-27 | H | $C_2H_5$ | $C_4H_9$ | $CF_3CH_2-$ | 126°C |
| A-28 | $C_2H_5$ | $C_2H_5$ | $C_4H_9$ | $CF_3CH_2-$ | (1,4855) |
| A-29 | H | $C_3H_5$ * | $C_4H_9$ | $CF_3CH_2-$ | 106 - 107°C |
| A-30 | H | $i-C_3H_7$ | H | $CF_3-CH-$<br>$CH_3$ | 185 - 186°C |
| A-31 | H | $C_3H_7$ | H | $CF_3-CH-$<br>$CH_3$ | 122 - 125°C |
| A-32 | H | $C_2H_5$ | H | $CF_3CH-$<br>$CH_3$ | 148 - 149°C |
| A-33 | $C_2H_5$ | $C_2H_5$ | H | $CF_3CH-$<br>$CH_3$ | 68 - 72°C |
| A-34 | H | $C_4H_9$ | H | $CF_3CH-$<br>$CH_3$ | 117 - 120°C |

Tabelle 1 (Fortsetzung)

| Bsp.-Nr. | $R^1$ | $R^2$ | $R^3$ | $R^4$ | Schmelzpunkt (Brechungsindex $n_D^{20}$) |
|---|---|---|---|---|---|
| A-35 | H | $CH_3$ | H | $CF_3CH-$<br>$CH_3$ | 150°C |
| A-36 | $CH_3$ | $CH_3$ | H | $CF_3CH-$<br>$CH_3$ | 120 - 123°C |
| A-37 | H | $t-C_4H_9$ | H | $CF_3CH-$<br>$CH_3$ | 141 - 146°C |
| A-38 | H | H | H | $CF_3CH-$<br>$CH_3$ | 135°C |
| A-39 | H | $CH_3$ | H | $CF_3CH_2CH_2-$ | 248 - 250°C |
| A-40 | $CH_3$ | $CH_3$ | H | $CF_3CH_2CH_2-$ | 200 - 202°C |
| A-41 | H | $C_2H_5$ | H | $CF_3CH_2CH_2-$ | 215 - 218°C |
| A-42 | $C_2H_5$ | $C_2H_5$ | H | $CF_3CH_2CH_2-$ | 130 - 132°C |
| A-43 | H | $n-C_3H_7$ | H | $CF_3CH_2CH_2-$ | 225 - 228°C |
| A-44 | H | $C_3H_5$* | H | $CF_3CH_2CH_2-$ | 205°C |
| A-45 | H | $i-C_4H_9$ | H | $CF_3CH_2CH_2-$ | 216 - 220°C |
| A-46 | H | $i-C_4H_9$ | H | $CF_3CH_2CH_2-$ | 141 - 148°C |

*$C_3H_5$ = $CH_2=CH-CH_2-$ (Allyl)

Tabelle 1 (Fortsetzung)

| Bsp.-Nr. | $R^1$ | $R^2$ | $R^3$ | $R^4$ | Schmelzpunkt (Brechungsindex $n_D^{20}$) |
|---|---|---|---|---|---|
| A-47 | H | $CH_2=CH-CH_2-$ | H | $CF_3CH(CH_3)-$ | 122 - 125°C |
| A-48 | H | $C_2H_5SCH_2CH_2-$ | H | $CF_3CH(CH_3)-$ | 101 - 104°C |
| A-49 | H | $CH_3OCH_2CH_2-$ | H | $CF_3CH(CH_3)-$ | 96 - 99°C |
| A-50 | $C_3H_7-$ | $C_3H_7-$ | H | $CF_3CH(CH_3)-$ | 82 - 84°C |
| A-51 | $i-C_3H_7-$ | $i-C_3H_7-$ | H | $CF_3CH(CH_3)-$ | (1.4953) |
| A-52 | H | $C_2H_5CH(CH_3)-$ | H | $CF_3CH(CH_3)-$ | 203°C |
| A-53 | H | $ClCH_2CH_2-$ | H | $CF_3CH(CH_3)-$ | 142 - 144°C |

Tabelle 1 (Fortsetzung)

| Bsp.-Nr. | $R^1$ | $R^2$ | $R^3$ | $R^4$ | Schmelzpunkt (Brechungsindex $n_D^{20}$) |
|---|---|---|---|---|---|
| A-54 | H | $i-C_4H_9-$ | H | $CF_3\underset{CH_3}{\underset{\vert}{C}}H-$ | 148 - 149°C |
| A-55 | H | $(CH_3)_3C-CH_2-$ | H | $CF_3\underset{CH_3}{\underset{\vert}{C}}H-$ | 152 - 154°C |
| A-56 | $CH_2=CH-CH_2-$ | $CH_2=CH-CH_2-$ | H | $CF_3\underset{CH_3}{\underset{\vert}{C}}H-$ | 55 - 60°C |
| A-57 | H | $HOCH_2CH_2-$ | H | $CF_3\underset{CH_3}{\underset{\vert}{C}}H-$ | 123 - 126°C |
| A-58 | H | $CH_3OCH_2CH_2CH_2-$ | H | $CF_3\underset{CH_3}{\underset{\vert}{C}}H-$ | 120 - 121°C |
| A-59 | H | $C_2H_5C(CH_3)_2-$ | H | $CF_3\underset{CH_3}{\underset{\vert}{C}}H-$ | 160 - 161°C |
| A-60 | H | $C_2H_5O\underset{O}{\underset{\Vert}{C}}-CH_2-$ | H | $CF_3\underset{CH_3}{\underset{\vert}{C}}H-$ | 139 - 141°C |

Beispiel A-35 / bevorzugte Verfahrensvariante

Es hat sich als besonders vorteilhaft erwiesen, die Verbindung des Beispiels A-35, ausgehend von Cyanurchlorid, in einer "Eintopfreaktion" herzustellen, wobei das Zwischenprodukt (s. Beispiel C-7) nicht isoliert wird. Im einzelnen arbeitet man zweckmäßigerweise wie folgt:

520 g (2,82 Mol) Cyanurchlorid werden bei Raumtemperatur in Toluol gelöst; die Lösung wird auf 5-10°C gekühlt. Unter Rühren läßt man hierzu bei 8-10°C 324 g (2,86 Mol) 1-Methyl-2,2,2-trifluor-ethylamin (s. Beispiel D-1) zutropfen, rührt kurze Zeit (ca. 15 Minuten) nach und gibt dann bei der gleichen Temperatur eine Lösung von 118 g NaOH/400 ml Wasser zu. Nach Anstieg der Temperatur auf ca. 15°C werden bei 15-20°C 310 ml wäßrige, 32,5 %-ige Methylaminlösung (3,2 Mol $CH_3NH_2$) zugegeben.

Man rührt dann wiederum 15 Minuten lang nach und tropft dann bei gleicher Temperatur 113 g NaOH/300 ml Wasser zu.

Dabei wird das Reaktionsgut sehr breiig. Nach kurzzeitigem Rühren bei 25°C wird mit HCl angesäuert und auf etwa 80°C erhitzt. Bei dieser Temperatur tritt Klarlösung ein. Man trennt die beiden Phasen, filtriert evtl. die heiße Toluollösung von wenig Unlöslichem und kühlt ab. Das Produkt kristallisiert in der Kälte bei 10 - 15°C aus der Toluollösung aus. Man saugt ab, wäscht mit Toluol und trocknet. Ausbeute:
588g (≙ 82 % der Theorie) 2-Chlor-4-methylamino-6-(1-methyl-2,2,2-trifluor-ethylamino)-s-triazin der Formel

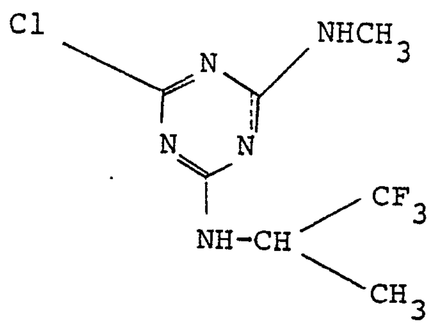

(A-35),

Schmelzpunkt 149 - 150°C ; gaschromatographisch bestimmte Reinheit: 99,8 %.

B) Triazine der Formel (Ib) - nach Verfahren A/2. Stufe

Beispiel B-1

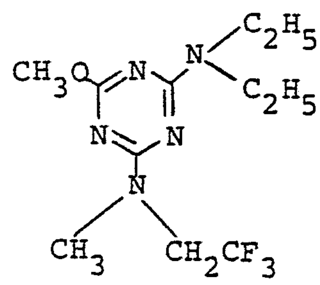

10,4 g (0,035 Mol) 2-Chlor-4-diethylamino-6-(N-methyl-N-2,2,2-trifluorethylamino)-s-triazin (Beispiel A-14) werden in 100 ml Methanol gelöst und mit 7 ml einer 1-normalen Natriummethylatlösung versetzt. Man erhitzt 1 Stunde zum Sieden, setzt Methylenchlorid zu und schüttelt die Reaktionslösung mehrmals mit Wasser aus und engt die Methylenchloridphase ein. Hierbei erhält man das Reaktionsprodukt in Form eines Öls. Ausbeute 7,6 g (= 75 % d.Th.) 2-Methoxy-4-diethylamino-6-(N-methyl-N-2,2,2-trifluorethylamino)-s-triazin; $n_D^{20}$: 1,4762.

In analoger Weise erhält man die in der nachfolgenden Tabelle 2 aufgeführten Verbindungen der Formel Ic:

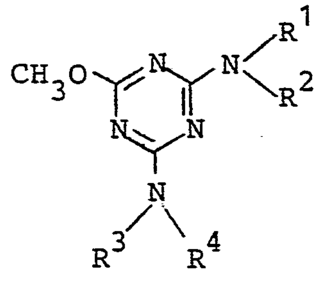

(Ic)

Tabelle 2

| Bsp.-Nr. | $R^1$ | $R^2$ | $R^3$ | $R^4$ | Schmelzpunkt (Brechungsindex $n_D^{20}$) |
|---|---|---|---|---|---|
| B-2 | $C_2H_5$ | $C_2H_5$ | $C_2H_5$ | $CF_3CH_2-$ | (1,4754) |
| B-3 | H | $i-C_3H_7$ | $CH_3$ | $CF_3CH_2-$ | 87 - 90°C |
| B-4 | H | $i-C_3H_7$ | $C_2H_5$ | $CF_3CH_2-$ | 76 - 77°C |

Beispiel B-5

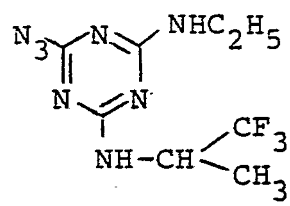

Eine Lösung von 7,8 g (0,029 Mol) 2-Chlor-4-ethylamino-6-(1-methyl-2,2,2-trifluorethyl-amino)-s-triazin (Beispiel A-32) und 2 g (0,031 Mol) Natriumazid ($NaN_3$) in 100 ml Dimethylformamid wird 3 Stunden lang auf dem siedenden Wasserbad erhitzt. Nach dem Erkalten gießt man das Reaktionsgemisch in Wasser und saugt das Reaktionsprodukt ab. Man wäscht es mit Wasser nach und trocknet es. Ausbeute 7 g (≙ 100 % d.Th.) 2-Azido-4-ethylamino-6-(1-methyl-2,2,2-trifluoroethyl-amino)-s-triazin; Schmelzpunkt 69 - 75°C.

C) Triazine der Formel (II) - nach Verfahren B

Beispiel C-1

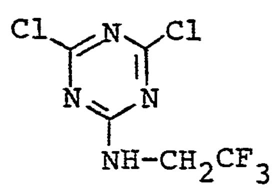

166 g (0,9 Mol) Cyanurchlorid werden in 1 l Aceton warm gelöst, von wenig Unlöslichem abfiltriert

und unter Rühren und Eiskühlung bei 0 - 5°C tropfenweise mit einer Lösung von 90 g (0,9 Mol) 2,2,2-Trifluorethylamin in 101 g (1,0 Mol) Triethylamin versetzt. Man rührt eine Zeitlang, setzt ca. 1 l Wasser zu und saugt das gebildete Kristallisat ab. Nach dem Trocknen erhält man 100 g (≙ 45 % d.Th.) 2,4-Dichlor-6-(2,2,2-trifluorethylamino)-s-triazin, Schmelzpunkt 115 - 118°C.

In analoger Weise lassen sich die in der folgenden Tabelle 3 aufgeführten Verbindungen der Formel (II) herstellen:

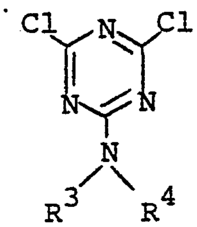

(II)

Tabelle 3

| Bsp. Nr. | $R^3$ | $R^4$ | Schmelzpunkt (Brechungsindex $n_D^{20}$) |
|---|---|---|---|
| C-2 | H | $CH_3CF_2CH_2$- | 104 - 105°C |
| C-3 | $CH_3$- | $CF_3$-$CH_2$- | 45 - 48°C |
| C-4 | $C_2H_5$- | $CF_3$-$CH_2$- | 70 - 72°C |
| C-5 | $C_4H_9$- | $CF_3$-$CH_2$- | (1,4882) |
| C-6 | H | $CF_3$-$CH_2$-$CH_2$- | 76 - 81°C |
| C-7 | H | $CF_3$-CH($CH_3$)- | (1,5026) |

D) Fluorhaltige Amine der Formel (V)

Beispiel D-1

$$CF_3-\underset{|}{CH}-CH_3$$
$$NH_2$$

(a) $CF_2=CH-CH_3 + HNO_3 + HF \rightarrow CF_3-\underset{|}{CH}-CH_3$
$$NO_2$$

1400 ml Fluorwasserstoff (wasserfrei) und 320 g Salpetersäure (konz.) werden in einem Stahlrührautoklaven vorgelegt und auf -30° bis -40°C gekühlt. Bei dieser Temperatur werden 390 g (5 Mol) 1,1-Difluorpropen eingeleitet. Man läßt auf Raumtemperatur aufwärmen, rührt 6 Stunden nach und gießt das Reaktionsgemisch dann auf 2 kg Eis. Die wäßrige Phase wird dreimal mit je 250 ml Dichlormethan extrahiert, die vereinigten organischen Phasen werden mit $NaHCO_3$-Lösung und Wasser neutral gewaschen, mit $MgSO_4$ getrocknet und destilliert. Nach Abtrennen des Lösungsmittels und eines Vorlaufes (25 g; Siedebereich: 43 - 98°C) erhält man 615 g (≙86 % d.Th.) 1-Methyl-2,2,2-trifluornitroethan /≙ 1-(Trifluormethyl)-nitroethan7 ; Siedepunkt (Kp) 98 - 100°C; $n_D^{20}$: 1,3380.

(b) 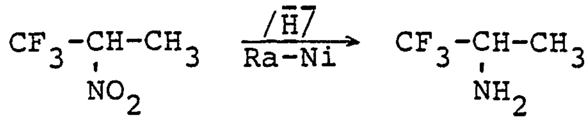


715 g (5 Mol) 1-Methyl-2,2,2-trifluor-nitroethan werden in 3 Liter Methanol mit 30 g Raney-Nickel bei einer Temperatur von 30 - 40°C und einem Druck von 40 bar hydriert. Nach dem Abfiltrieren des Katalysators wird mit konz. Salzsäure angesäuert und die flüchtigen Bestandteile werden abdestilliert. Zum Rückstand gibt man 600 g 50 %ige Natronlauge und destilliert das Reaktionsprodukt ab. Man erhält 520 g (≙ 92 % d.Th.) 1-Methyl-2,2,2-trifluor-ethylamin /≡ 1-(Trifluormethyl)-ethylamin7; Siedepunkt 47 - 48°C; $n_D^{20}$: 1,3215.

Beispiel D-2

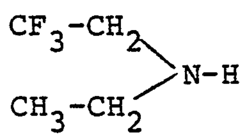

(a) $CF_3-CH_2-NH_2 + CH_3CHO \longrightarrow CF_3-CH_2-N{=}CH-CH_3$

Zu 99 g (1 Mol) 2,2,2-Trifluorethylamin werden innerhalb von 60 Minuten unter Eiskühlung 44 g (1 Mol) frisch destillierter Acetaldehyd zugetropft. Man läßt 1 Stunde nachrühren, gibt 15 g festes Kaliumhydroxid hinzu und trennt die Phasen. Die organische Pha-e wird über etwa

5 g festem Kaliumhydroxid getrocknet und anschließend über eine Kolonne destilliert. Nach einem Vorlauf (20 g; Kp: 18 - 42°C), der aus nicht umgesetztem Amin und Acetaldehyd besteht, erhält man 77,5 g (62 % der Theorie) an Ethyliden-2,2,2-trifluorethyl-amin.

Kp = 73 - 74°C

$n_D^{20}$ = 1,3415

(b) 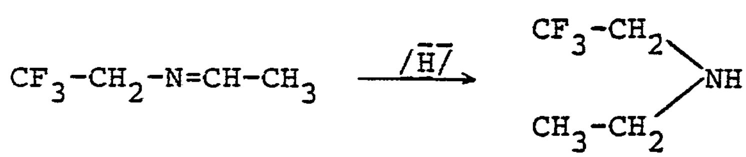


125 g (1 Mol) Ethyliden-2,2,2-trifluorethylamin werden in 250 ml absolutem Ethanol gelöst, mit 4 g Platin auf Kohle (5 %ig) versetzt und bei 30°C unter einem Wasserstoffdruck von 10 bar 90 Minuten lang hydriert. Nach dem Abfiltrieren des Katalysators wird mit konzentrierter Salzsäure angesäuert und unter vermindertem Druck bis zur Trockne eingeengt. Das dabei anfallende Produkt wird mit 100 ml 50 %iger wäßriger Natronlauge versetzt und destilliert. Man erhält auf diese Weise 83 g (65 % der Theorie) an 2,2,2-Trifluor-ethyl-N-ethylamin.

Kp = 61 - 62°C

$n_D^{20}$ = 1,3335

In analoger Weise werden auch die folgenden fluorierten Amine erhalten:

Beispiel D-3

(a) Zwischenprodukt:

$$CF_3-CH_2-N=CH-CH_2CH_3$$

Ausbeute: 64 % der Theorie

Kp = 93 - 95°C

(b) 2,2,2-Trifluor-ethyl-N-n-propylamin

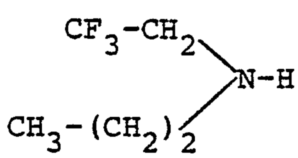

Ausbeute: 66 % der Theorie

$n_D^{20}$ = 1,3462

Kp = 74°C

Beispiel D-4

(a) Zwischenprodukt:

$$CF_3-CH_2-N=CH-CH_2-CH_2-CH_3$$

Ausbeute: 72 % der Theorie

Kp = 116 - 118°C

(b) 2,2,2-Trifluor-ethyl-N-n-butylamin

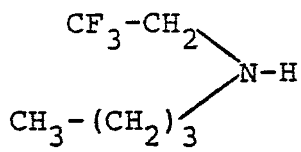

Ausbeute: 72 % der Theorie
$n_D^{20}$ = 1,3590
Kp = 84°C

Verwendungsbeispiele

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Bei diesem Test zeigen z.B. die folgenden Verbindungen der Herstellungsbeispiele eine ausgezeichnete Wirksamkeit: Nr. A-32, A-35.

## Beispiel B

Post-emergence-Test / Gewächshaus

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die gewünschten Wirkstoffmengen pro Flächeneinheit ausgebracht werden. Die Konzentration der Spritzbrühe wird so gewählt, daß in 2000 l Wasser/ha die in der Tabelle angegebenen Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Bei diesem Test zeigen z.B. die folgenden Verbindungen der Herstellungsbeispiele eine ausgezeichnete Wirksamkeit: Nr. A-32, A-35.

Patentansprüche

1) Fluorhaltige 4,6-Diamino-s-triazine der allgemeinen Formel I

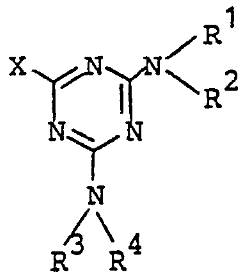

(I)

worin

X für Chlor, Alkoxy mit 1 - 4 C-Atomen, Alkylthio mit 1 - 4 C-Atomen oder den Azidrest steht,

$R^1$, $R^2$ und $R^3$ gleich oder verschieden sind und für Wasserstoff und/oder einen gesättigten oder ungesättigten aliphatischen Rest stehen und

$R^4$ für einen Fluoralkyl- oder Fluorchloralkylrest steht.

2) Fluorhaltige 4,6-Diamino-s-triazine der allgemeinen Formel (I) gemäß Anspruch 1, worin

X für Chlor, Alkoxy mit 1 - 4 C-Atomen, Alkylthio mit 1 - 4 C-Atomen oder den Azidrest steht, .

$R^1$, $R^2$ und $R^3$ jeweils unabhängig voneinander für Wasserstoff, Alkyl mit 1 - 6 C-Atomen oder Alkenyl mit 3 - 6 C-Atomen stehen,

$R^2$ zusätzlich für Ethylthioethyl, Methoxyethyl, 2-Chlorethyl, 2-Hydroxyethyl oder Ethoxycarbonylmethyl steht und

$R^4$ für einen Fluoralkylrest mit 1 - 8 C-Atomen und 1 - 9 F-Atomen oder einen Fluorchloralkylrest mit 1 - 8 C-Atomen und bis zu insgesamt 9 F- und Cl-Atomen steht.

3) 2-Chlor-4-ethylamino-6-(1-methyl-2,2,2-trifluorethylamino)-s-triazin der Formel

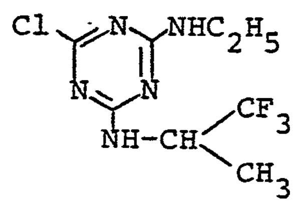

(A-32)

gemäß Anspruch 1.

4) 2-Chlor-4-methylamino-6-(1-methyl-2,2,2-trifluorethylamino)-s-triazin der Formel

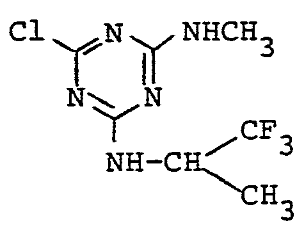

(A-35)

gemäß Anspruch 1.

5) Verfahren zur Herstellung von fluorhaltigen 4,6-Diamino-s-triazinen der allgemeinen Formel I

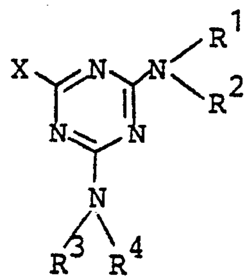

(I),

worin

X für Chlor, Alkoxy mit 1 - 4 C-Atomen, Alkylthio mit 1 - 4 C-Atomen oder den Azidrest steht,

$R^1$, $R^2$ und $R^3$ gleich oder verschieden sind und für Wasserstoff und/oder einen gesättigten oder ungesättigten aliphatischen Rest stehen und

$R^4$ für einen Fluoralkyl- oder Fluorchloralkylrest steht,

dadurch gekennzeichnet, daß man 2,4-Dichlor-6-(fluor-alkylamino)-s-triazine der allgemeinen Formel II

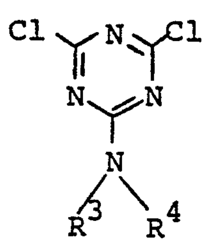

(II),

worin

$R^3$ und $R^4$ die oben angegebene Bedeutung haben,

mit einem Amin der Formel III

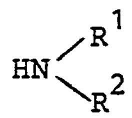

(III),

worin

$R^1$ und $R^2$ die oben angegebene Bedeutung haben,

in Gegenwart eines säurebindenden Mittels und gegebenenfalls in Gegenwart eines Verdünnungsmittels umsetzt (1. Stufe) und die hierbei gebildeten 2-Chlor-4,6-diamino-s-triazine der Formel Ia

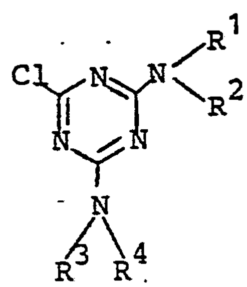

(Ia)

worin

$R^1$, $R^2$, $R^3$ und $R^4$ die oben angegebene Bedeutung haben,

gegebenenfalls mit einem $C_{1-4}$-Alkohol, einem $C_{1-4}$-Mercaptan oder mit Stickstoffwasserstoffsäure ($HN_3$) jeweils in Salzform oder in Gegenwart eines säurebindenden Mittels und gegebenenfalls in Gegenwart

eines Verdünnungsmittels umsetzt (2. Stufe), wobei 2-substituierte 4,6-Diamino-s-triazine der Formel Ib

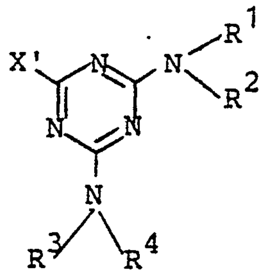

(Ib)

worin

X' für Alkoxy oder Alkylthio mit jeweils 1 - 4 C-Atomen oder für den Azidrest steht und

$R^1$, $R^2$, $R^3$ und $R^4$ die oben angegebene Bedeutung haben,

erhalten werden.

6) 2,4-Dichlor-6-(fluor-alkylamino)-s-triazine der allgemeinen Formel II

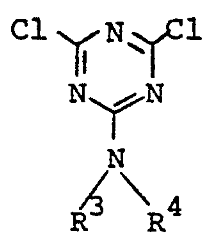

(II),

worin

$R^3$ und $R^4$ die oben angegebene Bedeutung haben.

7) Verfahren zur Herstellung von 2,4-Dichlor-6-(fluoralkylamino)-s-triazinen der allgemeinen Formel II

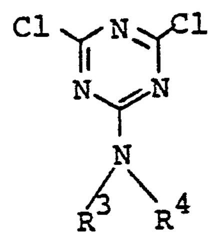

(II),

worin

$R^3$ und $R^4$ die oben angegebene Bedeutung haben,

dadurch gekennzeichnet, daß man Cyanurchlorid der Formel IV

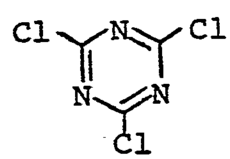

(IV)

mit fluorhaltigen primären und sekundären Aminen der Formel V

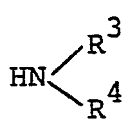

(V)

worin

$R^3$ und $R^4$ die oben angegebene Bedeutung haben,

in Gegenwart eines säurebindenden Mittels und gegebenenfalls in Gegenwart eines Verdünnungsmittels umsetzt.

8) Herbizide Mittel, gekennzeichnet durch einen Gehalt an mindestens einem fluorhaltigen 4,6-Diamino-s-triazin der Formel (I) gemäß Anspruch 1 bzw. 5.

9) Verwendung von fluorhaltigen 4,6-Diamino-s-triazinen der Formel (I) gemäß Anspruch 1 bzw. 5 zur Bekämpfung von Unkräutern.

10) Verfahren zur Herstellung von herbiziden Mitteln, dadurch gekennzeichnet, daß man fluorhaltige 4,6-Diamino-s-triazine der Formel (I) gemäß Anspruch 1 bzw. 5 mit Streckmitteln und/oder oberflächenaktiven Mitteln vermischt.